(19) Europäisches Patentamt
European Patent Office
Office européen des brevets

(11) Numéro de publication: **0 117 186**
**B1**

# (12) FASCICULE DE BREVET EUROPÉEN

(45) Date de publication du fascicule du brevet: **16.12.87**

(21) Numéro de dépôt: **84400236.0**

(22) Date de dépôt: **03.02.84**

(51) Int. Cl.⁴: **C 07 B 37/00,** C 07 C 17/28 // C07C43/192, C07F7/08, C07D221/04, C08F8/48, C07C17/26

## (54) Procédé de préparation de dérivés du cyclopropane.

(30) Priorité: **18.02.83 FR 8302640**

(43) Date de publication de la demande: **29.08.84 Bulletin 84/35**

(45) Mention de la délivrance du brevet: **16.12.87 Bulletin 87/51**

(84) Etats contractants désignés: **AT BE CH DE FR GB IT LI LU NL SE**

(56) Documents cité:
**DE-A-1 668 530**
**GB-A-983 203**
**US-A-3 264 359**

(73) Titulaire: **RHONE- POULENC CHIMIE, 25, quai Paul Doumer, F-92408 Courbevoie Cédex (FR)**

(72) Inventeur: **Rivier, Georges, 22bis, rue des Essarts, F-69500 Bron (FR)**
Inventeur: **Soula, Gérard, 33, rue Nungesser, F-69330 Meyzieu (FR)**

(74) Mandataire: **Cazes, Jean- Marie, RHONE- POULENC INTERSERVICES Service Brevets Chimie 25, quai Paul Doumer, F-92408 Courbevoie Cédex (FR)**

EP 0 117 186 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention a pour objet un procédé de préparation de dérivés halogènes du cyclopropane par réaction d'un composé oléfinique avec un halogénocarbène, tel que décret ci-dessous.

On connait, dans l'art antérieur, cette réaction en catalyse par transfert de phase solide-liquide. Plus particulièrement, est décrite l'utilisation des éthers-couronnes comme agent de transfert de phase solide-liquide, par exemple dans Tetrahedron letters, vol. 21 p. 613 et suivantes - 1980, Synthesis 1977 p. 682 - 683, et Journal of the American Chemical Society 96, 17 (21 Août 1974) p. 5632-5633.

On connait aussi la mise en oeuvre, comme agents de transfert de phase, des composés du type "cryptands" et des ammoniums quaternaires (Tetrahedron Letters précité).

Ces trois types de procédés ne sont pas actuallement industrialisables.

En effet, les éthers-couronnes et les cryptands sont des composés dont la synthèse est très difficile à réaliser à une échelle industrielle. Da plus, leur prix de revient est tellement élevé qu'il est prohibitif pour une exploitation commerciale. Quant aux ammoniums quaternaires, ils se décomposent an milieu basique (milieu de la réaction objet de la présente demande) ce qui se traduit, bien entendu, par une parte de catalyseur et de rendement.

Il est aussi connu d'après le brevet GB 983.203 un procédé de préparation de dérivés du cyclopropane par réaction d'un mélange anhydre d'un hydroxyde alcalin, d'un haloforme et d'un accepteur de radicaux dihalocarbéniques, l'agent haloforme contenant au moins un atome de chlore ou de brome. Cette réaction est catalysée par l'utilisation de solvants à base d'éthers.

Le procédé selon l'invention pallie les inconvénients de l'art antérieur.

La présente invention a pour objet un procédé de préparation de dérivés halogénés du cyclopropane dans lequel on fait réagir un composé oléfinique, de formule:

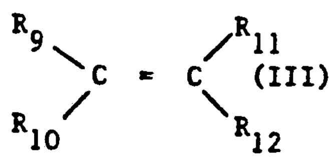

(III)

dans laquelle:

. $R_9$ représente un radical alkyle, alcényle, alkoxy, aryloxy, dialkylamino, diarylamino, alkylsulfure, arylsulfure, alkylpolysulfure, arylpolysulfure, alkylsilane ou arylsilane ayant de 1 à 100 atomes de carbone;

.$R_{19}$, $R_{11}$ et $R_{12}$, identiques ou différents, représentent un atome d'hydrogène, un radical alkyle de 1 à 25 atomes de carbone, ou un radical phényle, deux d'entre eux pouvant être liés de façon à former un cycle hydrocarboné de 4 à 12 atomes de carbone ou un hétérocycle comportant un atome d'oxygène ou d'azote et ayant de 4 à 7 atomes de carbone avec un dérivé halogéné de formule:

$X_1X_2X_3CH$ (I)

dans laquelle $X_1$, $X_2$ et $X_3$ identiques ou différents, représentent un atome d'halogène, l'un au moins étant un atome de chlore ou de brome, et avec une base alcaline organique ou minérale caractérisé en ce que la réaction est réalisée en présence d'un agent séquestrant da formule générale:

$N\text{-}[CHR_1\text{-}CHR_2\text{-}O\text{-}(CHR_3\text{-}CHR_3\text{-}CHR_4\text{-}O)_n\text{-}R_5]_3$ (II)

dans laquelle n est un nombre entier supériaur ou égal à 0 et inférieur ou égal à 10 ($0 \leqslant n \leqslant 10$), $R_1$, $R_2$, $R_3$ et $R_4$, identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $-C_mH_{2m}-Ø$ ou $C_mH_{2m+1}-Ø-$, où m est compris entre 1 et 12 ($1 \leqslant m \leqslant 12$) et en ce que le rapport molaire de l'agent sequestrant de formule (II) au composé oléfinique de formule (III) est de preference inferieur à 0,2 et plus preferentiellement ce rapport est compris entre 0,01 et 0,1.

La base alcaline minérale de préférence mise en oeuvre, répond à la fomule générale:

$M^+A^-$ (III)

dans laquelle $M^+$ représente un cation alcalin et $A^-$ représente $OH^-$ ou $NH_2^-$.

On peut citer comme exemples de bases alcalines minéales: la soude, la potasse, l'amidure de sodium, la lithine, l'amidure de potassium.

La base alcaline organique, de préférence mise en oeuvre, a pour formule:

$R_6(O^-)_p(M^+)_p$ (IV)

dans laquelle:

-M+ représente un cation alcalin

- p est supérieur ou égal à 1 et inférieur ou égal à 4

- quand p = 1, $R_6$ représente:

. un radical alkyle ayant de 1 à 18 atomes de carbone ou phényle éventuellement substitué par un radical alkyle avant de 1 à 12 atomes de carbone;

. un radical alkyle ayant de 2 à 5 atomes de carbone substitué par au moins un radical OH ou alkoxy ayant de 1 à 4 atomes de carbone;

. ou un radical de formule:

$R_7$-(O-$R_8$-)-$_t$

dans laquelle $R_7$ et $R_8$ représentent un radical hydrocarboné ayant de 2 à 4 aromes de carbone et t est supérieur ou égal à 1 et inférieur ou égal à 4;

- quand p est supérieur ou égal à 2 et inférieur ou égal à 4, $R_6$ représente:

. un radical hydrocarboné éventuellament substitué par au moins un radical OH, ayant de 2 à 5 atomes de carbone.

On peut citer comme exemples de base de formule IV quand p=1

- le méthylate de sodium, le méthylate de potassium, le tertiobutylate de sodium, le tertiobutylate de potassium, le laurate de sodium, l'éthylhexanoate de sodium, l'éthylhexanoate de potassium, le dodécylate de potassium, le phénate de sodium, le dodécylphénate de potassium;

- le butoxyéthanolate de sodium, le méthoxyéthanolate de sodium, l'éthoxypropylate de potassium, le monoglycolate de sodium, le monopentaerythritolate de sodium;

- le monoalcoolate de sodium de l'éther monométhylique du triéthylene glycol: $CH_3$-(O-$CH_2$-$CH_2$)$_3$-O$^-$Na$^+$, le monoalcoolate de pocassium de l'éther monoéthylique du dipropylène glycol:

[$C_2H_5$-($OCH_2CHCH_3$)$_2$-O$^-$K$^+$].

On peut citer comme exemples de formule IV quand 2 < p< 4: le dialcoolate de sodium du butanediol-1,4: Na$^+$$^-$O-($CH_2$)$_4$-O$^-$Na$^+$, le trialcoolate de potassium du pentaérythritol:

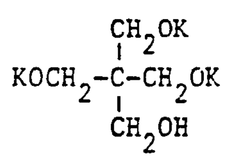

Selon un mode de réalisation préférentiel de l'invantion, on utilise un agent séquestrant de formule (II) dans laquelle $R_1$, $R_2$, $R_3$ et $R_4$ représentent un atome d'hydrogène ou un radical méthyle, $R_5$ et n ayant la signification précédente.

Parmi ces derniers, on préfère encore plus particulièrement mettre en oeuvre les agents séquestrants pour lesquels n est supérieur ou égal à 0 et inférieur ou égal à 6 et pour lesquels $R_5$ représente un radial alkyle ayant de 1 à 4 atomes de carbone.

On peut citer

- la tris (oxa-3 butyl)amine de formule:

N-($CH_2$-$CH_2$-O-$CH_3$)$_3$

- la tris (dioxa-3,6 heptyl)amine de formule:

N-$CH_2$-$CH_2$-O-$CH_2$-$CH_2$-O-$CH_3$)$_3$

- la tris (trioxa-3,6,9 déptyl)amine de formule:

N-($CH_2$-$CH_2$-O-$CH_2$-$CH_2$-O-$CH_2$-$CH_2$-O-$CH_3$)$_3$

- la tris (dioxa-3,6 octyl)amine de formule:

N-($CH_2$-$CH_2$-O-$CH_2$-$CH_2$-O-$C_2$-$H_5$)$_3$

- la tris (trioxa-3,6,9 undécyl)amine de formule:

N-($CH_2$-$CH_2$-O-$CH_2$-$CH_2$-O-$CH_2$-$CH_2$-O-$C_2H_5$)$_3$

- la tris(dioxa-3,6 nonyl)amine de formule:

N-($CH_2$-$CH_2$-O-$CH_2$-$CH_2$-O-$C_3H_7$)$_3$

- la tris(trioxa-3,6,9 dodécyl)amine de formule:

N-($CH_2$-$CH_2$-O-$CH_2$-$CH_2$-O-$CH_2$-$CH_2$-O-$C_3H_7$)$_3$

- la tris(dioxa-3,6 décyl)amine de formule:

N-($CH_2$-$CH_2$-O-$CH_2$-$CH_2$-O-$C_4H_9$)$_3$

- la tris(trioxa-3,6,9 tridécyl)amine de formule:

N-($CH_2$-$CH_2$-O-$CH_2$-$CH_2$-O-$CH_2$-$CH_2$-O-$C_4H_9$)$_3$

- la tris(tétra-oxa-3,6,9,12 tridécyl)amine de formule:

N-[$CH_2$-$CH_2$-O-($CH_2$-$CH_2$-O-$_3$)-$CH_3$]$_3$

- la tris(hexa-oxa-3,6,9,12,15,18 nonadécyl)amine de formule:

N-[$CH_2$-$CH_2$-O-($CH_2$-$CH_2$-O)-$_5$-$CH_3$]$_3$

- la tris(dioxa-3,6 méthyl-4 heptyl)amine de formule:

$N-[CH_2-CH_2-OCH-(CH_3)-CH_2-O-CH_3]_3$

- la tris(dioxa-3,6 diméthyl-2,4 heptyl) amine de formule:

$N-[CH_2-CH-(CH_3-OCH(CH_3)-CH_2-O-CH_3]_3$

Les amines de formule II peuvent être préparées comme décrit dans le brevet français publié sous le numéro 2 450 120.

Au sens de la présente invention, on entend par composé oléfinique tout composé organique non aromatique comportant au moins une double liaison. L'oléfine peut être porteuse de groupements fonctionnels ou non. L'invention vise le cas où une seule double liaison réagit avec le dérivé halogéné comme la cas où plusieurs doubles liaisons réagissent.

Les composés oléfiniques qui peuvent être mis en oeuvre dans le cadre de la présente invention ont, de préférence, la formule générale:

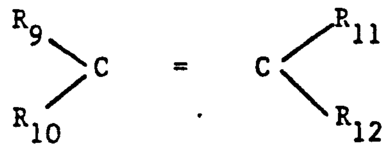 (III)

dans laquelle:

. $R_9$ représente un radical alkyle, alcényle, alkoxy, aryloxy, dialkylamino, diarylamino, alkylsuliure, arylsulfure, alkylpolysulfure, arylpolysulfure, alkylsilane ou arylsilane avant de 1 à 100 atomes de carbone;

. $R_{10}$, $R_{11}$ et $R_{12}$, identiques ou différents, représentent un atome d'hydrogène, un radical alkyle ayant au moins 1 atome de carbone ou un radical phényle, deux d'entre eux pouvant être liés de façon à former un cycle hydrocarboné de 4 à 12 atomes de carbone ou un hétérocycle comportant un atome d'oxygène ou d'azote et ayant de 4 à 7 atomes de carbone.

Le radical peut contenir un nombre très élevé d'atomes de carbone. Il peut être un radical ayant une masse allant jusqu'à 1.000.000.

On peut citer comme exemples de composés oléfiniques pouvant être mis en oeuvre dans le cadre du procédé selon l'invention: les hexènes, triméthylpentènes, diméthylbutènes, cyclohexènes, tétramère du propylène, polyisobutène, limonène, cyclooctatétraène, cyclododécatriène, éthylvinyléther, chlorure d'allyle, triméthylsilylvinyléther, pyrrole, furanne.

On peut citer comme exemples de composés halogénés de formule I le trichlorométhane, le tribromométhane, le bromodichlorométhane, le bromodifluorométhane, le chlorodibromométhane, le bromodiiodométhane, le bromochlorofluorométhane.

Le choix de l'agant séquestrant le plus adapté à la mise en oeuvre du procédé selon l'invention se fait en tenant compte de la taille du cation de la base alcaline mise en oeuvre. Plus la taille du cation sera importante, plus la nombre d'atomes d'oxygène contenus dans la molécule de l'agent séquestrant devra être élevé.

C'est ainsi que lorsque l'on utilisera le sodium, on préférers utiliser comme agent séquestrant la tris dioxa-3,6 heptyl amine.

Les dérivés du cyclopropané obtenus par le procédé selon l'invention ont pour formule générale:

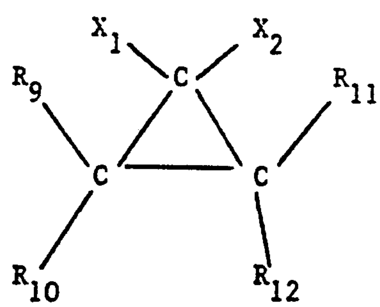 (IV)

On peut citer comme exemples de tels composés: le n-butyl-2 chloro-1 fluoro-1 cyclopropane, le t-butyl-2 dibromo-1,1, diméthyl-3,3 cyclopropane, le diodo-1,1 diméthyl-2,3 cyclopropane, le dichloro-2,2 bicyclo[$O_{1-3}$] heptane, les alkyl dibromocyclopropanes, les alkyls dichlorocyclopropanes, le méthyl-2 méthylcyclohexenyl-2 dibromo-1,1 cyclopropane, le tétrachloro-2,2,7,7 tricyclo [$O_{1.3}O_{6.8}$] tétradécène, l'hexachloro-2.2, 7.7, 12.12, le tétracyclopentadécane[$O_{1.3}O_{6.8}O_{11.13}$] le dichloro-1,1 éthoxy-2 cyclopropane, le dibromo-1,1 chlorométhyl-2 cyclopropane, le dichloro-1,1 triméthylsilyl-2 cyclopropane, la métachloropyridine, le dichloro-6,6 bicyclo [$O_{1.5}$]oxa-2 hexane.

Ils sont intéressants notamment comme intermédiaires dans le domaine des additifs pour la lubrification et, pour la synthèse d'halogénopyridines, utiles dans le domaine pharmaceutique at phytosanitaire.

Le dérivé halogéné de formule I est utilisé de préference en quantité égale à au moins 0,2 fois la stoechiométrie par rapport au nombre de double liaisons et encore plus préférentiellement comprise entre 0,5 et 3.

Le nombre de moles de base alcaline mises en oeuvre est de préférence d'au moins 2 fois la stoechiométrie par rapport au nombre de double liaisons et, encore plus préférentiellement, comprise entre 3 et 5.

On peut opérer en l'absence ou en présence de solvant. Lorsqu'on en utilise un, il est choisi de préférance parmi: les solvants aproriques apolaires ou peu polaires. On peut citer comme exemples de tels solvants: l'hexane, l'octane, le benzène, le toluène, les chlorobenzènes, le diéthyléther, le rétrahydrofuranne, le 1.2 diméthoxyéthane, le dichloroéthane.

Lorsqu'on opère sans solvant, c'est le composé de formule I qui joue le r*ole de solvant.

La température de réaction est comprise de préférence entre -20°C et 200°C. Encore plus préférentiellement, on opère entre 15 et 110°C.

La pression dépend de la nature du composé halogéné. On opère généralement sous pression atmosphérique avec les composés non fluorés et sous pression avec les composés fluorés.

Le procédé selon l'invention peut être représenté comme suit (dans le cas d'une base minerale):

$X_1X_2X_3CH + M^+A^- \rightarrow (X_1X_2X_3C^-M^+)\ N[CHR_1\text{-}CHR_2\text{-}O\text{-}(CHR_3\text{-}CHR_4\text{-}O)_n\text{-}R_5]_3$

Ce complexe se solubilise dans le milieu où se trouve le substrat:

$X_1X_2C\!: + (M^+X_3^-)\ N[CHR_1\text{-}CHR_2\text{-}O\text{-}(CHR_3\text{-}CHR_4\text{-}O)_n\text{-}R_5]_3$

$MX_3 + N[CHR_1\text{-}CHR_2\text{-}O\text{-}CHR_3\text{-}CHR_4\text{-}O)_n\text{-}R_5]_3$

L'agent séquestrant est libéré et peut retourner à la première étape:

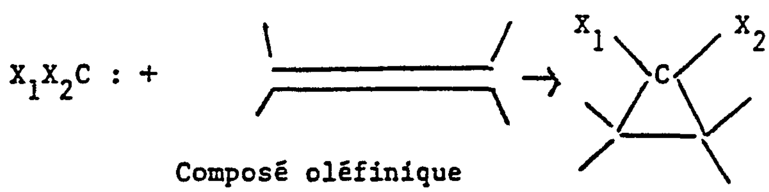


L'avantage prépondérant lié à l'utilisation des agents séquestrants selon l'invention réside dans le fait que avec les composés de formule II, on observe une séquestration préférentielle du cation lié au carbanion dérivé du composé de formule I ce qui permet sa solubilisation dans le milieu réactionnel. En effet, la séquestration de ce cation lié au carbanion est favorisée par rapport à la séquestration du cation lié à l'anion halogène. Cette sensibilité à la nature de l'anion est très marquée dans le cas des agents séquestrants de formule II alors qu'elle n'existe pas ou peu dans le cas des éthers-couronnes et des cryptands. Cette sélectivité rend toujours l'agent séquestrant de formule II plus efficace dans la réaction objet de la présente demande alors que, parfois, il peut avoir un pouvoir solubilisant moins élevé que les éthers-couronnes ou les agents séquestrants.

L'invention va être maintenant plus complètement décrite à l'aide des exemples qui vont suivre.

**Exemple 1**

Dans un ballon tricol muni d'un agitateur magnétique, surmonté d'une ampoule de coulée, d'un réfrigérant ascendant et d'un thermomètre, on charge 2,5 moles de solvant (280 g) de chlorobenzène anhydre - 0,5 mole d'héxène 1 (12 g) - 2 g de tris(dioxa-3,6 heptyl)amine et 60 g de soude finement divisée (1,5 mole).

Puis on coule très lentement 0,75 mole de chloroforme (90 g) an maintenant une très forte agitation dans le milieu. La réaction est exothermique. On maintient une température de 45°C durant 4 heures.

Le milieu est ensuite refroidi, filtré, puis lavé à l'eau pour éliminer les dernières traces de soude. La solution organique est séchée sur sulfate de sodium anhydre puis distillée (élimination du chlorobenzène et du chloroforme en excès).

On obtient le n-butyl dichlorocyclopropane de formule:

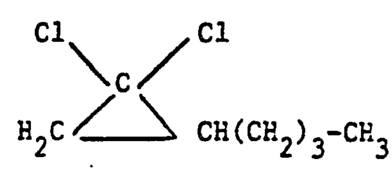

La taux de transformation est de 85 %.

**Exemple 2**

Dans les mêmes conditions opératoires que dans l'exemple 1 on charge 112 g de diisobutène (1 mole) contenant 80 % de triméthyl-2,4,4 pantène-1 et 20 % de triméthyl 2,4,4 pentène-2, 500 $cm^3$ de chlorobenzène, 3 moles de soude pulvérisée (120 g) et 6 g de tris(dioxa-3,6 heptyl)amine.

On coule ensuite 1,5 mole de bromoforme 379,5 g en maintenant une température de 55° C et une très forte agitation. Cette température est maintenue pendant 8 heures.

On obtient après traitement les deux dérivés de formules:

a) dibromo 11-méthyl-2 néopentyl-3 cyclopropane:

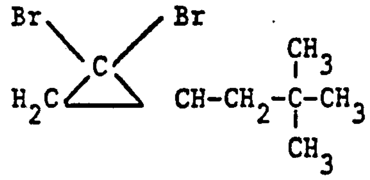

Le taux de transformation est de 80 %.

b) dibromo-1,1 diméthyl-2,2 t-bytyl-3 cyclopropane

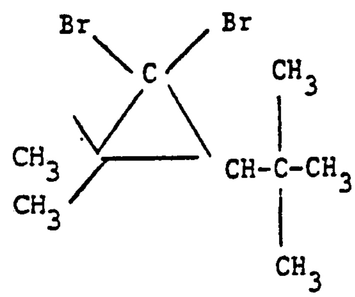

Le taux de transformation est de 80%.

**Exemple 3**

On charge 0,1 mole (16,8 g) de tétramère de propylène oléfine (ramifié en $C_{12}$), 56 g de chlorobenzène anhydre, 12 g (0,3 mole) de soude finement pulvérisée et 0,4 g de tris(dioxa-3,6 heptyl)amine.

Sous forte agitation on coule 18 g (0,15 mole) de chloroforme en maintenant une température de 55° C pendant 6 heures.

Après traitement l'analyse RMH montre qu'on obtient un mélange d'alkyl dichloro-1,1 cyclopropanes dont les chaînes alkyles représentent 10 atomes de carbone.

Le taux de transformation est de 85 %.

**Exemple 4**

On charge 47 g (0,05 mole) de polyisobutène de masse moyenne 950, 28 g de chlorobenzène anhydre (0,25 mole), 6 g de soude pulvérisée (0,15 mole) et 3,4 g de tris(dioxa-3,6 heptyl)amine.

Sous forte agitation on coule 8,9 g de chloroforme (0,055 mole) puis on maintient pendant 8 heures une température de 50° C.

Après traitement on obtient une huile constituée d'un mélange d'alkyl dichloro-1,1 cyclopropanes dont les chaînes alkyles représentent environ 65 atomes de carbone.

Le taux de transformation est de 75 %.

**Exemple 5**

On charge 328 g (2 moles) de 1,5,9-cyclododécatriène, 720 g (18 moles) de soude pulvérisée, 29 g de tris(3,6-dioxaheptylamine) et 2000 $cm^3$ de chlorobenzène anhydre puis on coule lentement 720 $cm^3$ soit 9 moles de

chloroforme (1075,5 g). On maintient le milieu réactionnel pendant 11 heures à 55°C. Après filtration et lavage du filtrat pour éliminer la soude, la phase organique est séchée sur sulfate de sodium anhydre.

Après distillation des solvents et des produits légers, le produit est distillé sous vide et on obtient 448 g d'un produit orange visqueux contenent:

. 70 % d'hexachloro-2.2,7.7,12.12 tétracyclo ($O_{1.3}O_{6.8}O_{11.13}$) pantadécane;

. 30 % de tétrachloro-2.2, 7.7 tricyclo ($O_{13}O_{6.8})_{11}$ tétradécène.

**Exemple 6**

On a chargé 855 g (0,9 mole) de polyisobutène, 500 cm$^3$ de chlorobenzène, 43 g de tris(trioxa-3,6,9 décyl)amine et 157 g (2,7 mole) de potasse broyée. On agite vigoureusement le milieu, puis on coule 342 g (1,35 mole) de bromoforme en maintenant une température de 55°C durant 8 heures.

Après filtration et distillation du solvant (chlorobenzène) et du bromoiorme en excès, on obtiant un produit très visqueux.

D'après l'analyse en RMN on peut affirmer que nous sommes en présence d'un mélange d'alkyl dibromo-11 cyclopropanes ayant au total environ 70 atomes de carbone.

Le taux de transformation est de 78 %.

**Exemple 7**

On charge 34 g de limonène (0,25 mole), 89,5 g de chloroforme (0,75 mole), 60 g (1,5 mole) de soude, 2,4 g de tris(dioxa-3,6 heptyl)amine et 140 g (1,25 mole) de chlorobenzène.

La mélange réactinnel est porté à 55°C pendant 6 heures après la fin da coulée du chloroforme.

Après traitement habituel on isole une huile (taux de transformation 75 %) contanant des dichlorocyclopropanes substitués par des chaînes alkyles ayant 8 atomes de carbone.

**Exemple 8**

On charge 41 g (0,5 mole) de cyclohexène, 28,1 g (0,25 mole) de chlorobenzène, 2 g de-tris(dioxa-3,6 heptyl)amine, 60 g (1,5 mole) de soude finement pulvérisée, puis on coule 89,6 g (0,75 mole) de chloroforme. Le mélange réactionnel est porté à 55°C pendent 6 heures.

On obtient le composé dichloro-2,2 bicyclo($O_{1.3}$)heptane avec un taux da transformation de 85 %. L'analyse RMN confirme la structure.

**Exemple 9**

On charge 7,65 g (0,1 mole) de chlorure d'allyle, 56 g de chlorobenzène anhydre, 12 g (0,3 mole) de soude finement pulvérisée et 0,5 g de tris(dioxa)3,6 heptyl)amine.

Sous forte agitation on coule 38 g (0,15 mole) de bronoforme; on maintient une température de 35°C pendant 10 heures.

Après traitement habituel on isole le chlorométhyl-2 dibromo-1,1 cyclopropane formé.

Le taux de transformation est de 70 %.

**Exemple 10**

On charge 7,2 g (0,1 mole) de vinyléthyléther, 56 g de chlorobenzène, 12 g (0,3 mole) d'amidure de sodium finement divisé et 0,4 g de tris(dioxa-3,6 heptyl)amine.

Sous forte agitation, on coule 18 g (0,15 mole) de chloroforme. Le mélange réactionnel est porté à 25°C pendant 40 heures.

On obtient ainsi le dichloro-1,1 éthoxy-2 cyclopropane avec un taux de transformation de 80 %.

**Exemple 11**

On opère comme dans l'exemple 1, mais dans un réacteur, compte-tenu de la volatilité du vinyltriméthylsilane.

On charge 10 g (0,1 mole) de triméthylvinylsilane, 56 g de chlorobenzène anhydre, 12 g (0,3 mole) de soude finement pulvérisée et 0,4 g de tris(dioxa-3,6 heptyl)amine.

Sous forte agitation on introduit par une pompe 18 g de chloroforme (0,15 mole). Le mélange réactionnel est porté à 45° C pendant 4 heures.

Après traitement on obtient le dichloro-1,1 triméthylsilyl-2 cyclopropane, avec un taux de transformation de 80 %.

**Exemple 12**

En opérant comme dans l'exemple 1, on charge 8,4 g (0,1 mole) de 2,3-diméthyl-2-butène, 56 g de chlorobenzène anhydre, 12 g (0,3 mole) de soude finement pulvérisée et 0,4 g de tris(dioxa-3,6 heptyl)amine.

Sous forte agitation on introduit 31,2 g (0,15 mole) de chlorodibromométhane.

On laisse se poursuivre la réaction à 55° C pendant 8 heures.

Après traitement, on obtient essentiellement du bromo chloro-1,1 tétraméthyl-2,2,3,3 cyclopropane.

Le taux de transformation en bromo chloro-1,1 tétraméthyl-2,2,3,3 cyclopropane est de 65 %.

**Exemple 13**

On charge 6,7 g (0,1 mole) de pyrrole fraichement distillé, 56 g de chlorobenzène anhydre, 12 g (0,3 mole) de soude finement pulvérisée et 0,4 g de tris(dioxa-3,6 heptyl)amine en maintenant une température de 15° C.

Sous forte agitation, on introduit 18 g de chloroforme en maintenant cette température à 15° C pendant 1 heure, puis on chauffe à 45° C pandant 3 heures. Le dichloro-2,2 aza-4 bicyclo ($O_{1,3}$) hexène se décompose immédiatement en chloro-3 pyridine.

La phase organique après filtration est distillée et une fraction est recueillie entre 136 et 170° C. Cette fraction analysée se compose de chlorobenzène, de chloropyridine et de produits non-identifiés.

Le taux de transformation en chloro-3 pyridine est de 19 %.

**Exemple 14**

On charge 10 g d'un polymère d'isobutène de masse moléculaire moyenne en poids d'environ 100.000 dans 500 g de chlorobenzène.

On porte à reflux pour accélérer la dissolution puis on refroidit à 30° C.

On ajoute au milieu réactionnel 1 g de soude pulvérisée, 0,5 g de tris(dioxa-3,6 heptyl)amine. Sous forte agitation on coule 3 g de bromoforme en maintenant une température de 55° C pendant 8 heures.

Après filtration, lavages à l'eau, on effectue une première évaporation à 120° C sous vide du solvant (chlorobenzène) er du bromoforme en excès.

Une deuxième évaporation sous grand vide ($10^{-3}$ mmHg) permet d'éliminer les dernières tracas de solvant.

L'analyse élémentaire du produit polymérique obtenu montre la présence de 0,11 % de brome et moins de 30 ppm de sodium. Une nouvelle évaporation en film sous grand vide $10^{-4}$ mmHg ne modifie pas la concentration en brome du produit confirmant la fixation du brome sur le polymère avec un taux de transformation d'environ 60 %.

**Exemple 15**

On prépare 30 g de T-butylate de potassium dans environ 50 g d'alcool t-butylique, puis on charge 60 g de chlorobenzène, 0,4 g de tris(dioxa-3,6 heptyl)amine et 9 g d'un mélange d'oléfines an $C_{22}$-$C_{26}$ (moyenne $C_{24}$).

Sous forte agitation on coule 9 g de chloroforme an maintenant la température à 55° C pendant 6 heures.

Après traitement on obtient un mélange d'alkyldichloro-1,1 cyclopropane en $C_{20}$-$C_{24}$ (moyenne $C_{22}$).

Le taux de transformation est de 85 %, structure confirmée par RMN.

**Exemple 16**

On prépare 10 g de méthoxyéthylate de sodium dans 60 g d'éther monométhylique du monoéthylèneglycol, on charge 60 g de chlorobenzène, 0,4 g de tris(dioxa-3,6 heptyl)amine er 5,6 g de 2-octène (0,05 mole).

Sous forte agitation on coule 20 g de bromoforme (0,07 mole) en maintenant une température de 50°C pendant 5 heures.

On obtient après traitement le dibromo-1,1 méthyl-2 pentyl-3 cyclopropane.

Le taux de transformation est de 80 %, structure confirmée par RMN.

**Revendications**

1. Procédé da préparation de dérivés halogénés du cyclopropane dans lequel on fait réagir un composé oléfinique, de formula:

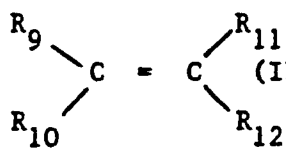

(III)

dans laquelle:

. $R_9$ représente un radical alkyle, alcényle, alkoxy, aryloxy, dialkylamino, diarylamino, alkylsulfure, arylsulfure, alkylpolysulfure, arylpolysulfure, alkylsilane ou arylsilane ayant de 1 à 100 atomes de carbone;

. $R_{10}$, $R_{11}$ et $R_{12}$, identiques ou différants, représentent un atome d'hydrogène, un radical alkyle de 1 à 25 atomes da carbone, ou un radical phényle, deux d'entre eux pouvant être liés de façon à former un cycle hydrocarboné de 4 à 12 atomes de carbone ou un hétérocycle comportant un atome d'oxygène ou d'azote et ayant de 4 à 7 atomes da carbone avec un dérivé halogéné da formule:

$X_1X_2X_3CH$ (I)

dans lequalle $X_1$, $X_2$ et $X_3$ identiques ou différents, représentent un atome d'halogène, l'un au moins étant un atome de chlore ou de brome, et avec une base alcaline organique ou minérale caractérisé en ce que la réaction est réalisée en présence d'un agent séquastrant de formule générale:

$N[CHR_1\text{-}CHR_2\text{-}O\text{-}(CHR_3\text{-}CHR_4\text{-}O)_n\text{-}R_5]_3$ (II)

dans laquelle n est un nombre entier supérieur ou égal à 0 et inférieur ou égal à 10 ($0 \leqslant n \leqslant 10$), $R_1$, $R_2$, $R_3$ et $R_4$, identiques ou différants représentent un atome d'hydrogène ou un radical alkyle ayant de 1 à 4 atomes de carbone et $R_5$ représente un radical alkyle ou cycloalkyle ayant de 1 à 12 atomes da carbone, un radical phényle ou un radical $-C_mH_{2m}-Ø-$, ou $C_mH_{2m+1}-Ø-$, où m est comprise entre 1 et 12 ($1 \leqslant m \leqslant 12$) et en ce que le rapport molaire de l'agent sequestrant de formule (II) au composé oléfinique de formule (III) est de preference inferieur à 0,2 et plus preferentiellement ce rapport est compris entre 0,01 et 0,1.

2. Procédé selon la revendication 1 caractérisé en ce que dans la formule II $R_1$, $R_2$, $R_3$ et $R_4$ représentent un atome d'hydrogène ou un radical méthyle, $R_5$ et n ayant la signification précédante.

3. Procédé selon la revendication 2 caractérisé an ce que dans la formule II n est supérieur ou égal à 0 et inférieur ou égal à 6 et $R_5$ représente un radical alkyle ayant de 1 à 4 atomes de carbone.

4. Procédé selon l'une quelconque das revendications précédentes, caractérisé en ce que la base alcaline minérale répond à la formule générale:

$M^+A^-$ (III)

dans laqualle $M^+$ représente un cation alcalin et $A^-$ représente $OH^-$ ou $NH_2$.

5. Procédé selon l'une quelconque des revendications 1 à 3, caractérisé en ce que la base alcaline organique a pour formule:

$R_6(O^-)_p(M^+)_p$ (IV)

dans laquelle:

- $M^+$ représente un cation alcalin
- p est supérieur ou égal à 1 et inférieur ou égal à 4
- quand p = 1, $R_6$ représente:

. un radical alkyle ayant de 1 à 18 atomes da carbone ou phényle éventuellement substitué par un radical

alkyle ayant de 1 à 12 atomes de carbone;

. un radical alkyle ayant da 2 à 5 atomes de carbone substitué par au moins un radical OH ou alkoxy ayant de 1 à 4 atomes de carbone;

. ou un radical de formule:

$R_7$-(O-$R_8$-$)_t$

dans laqualle $R_7$ at $R_8$ représentent un radical hydrocerboné ayant de 2 à 4 atomes de carbone et t est supériaur ou égal à 1 et inférieur ou égal à 4;

- quand p est supérieur ou égal à 2 et infériaur ou égal à 4, $R_6$ représente:

. un radical hydrocarboné éventuellement substitué par au moins un radical OH, ayant de 2 à 5 atomes de carbone.

6. Procédé selon l'une quelconque des revendication précédentes, caractérisé en ce que le dérivé halogéné de formule I et l'hydroxyde alcalin sont utilisés de préference en quantité égale à au moins 0,2 fois la stoechiométrie par rapport au nombre de double liaisons et encore plus préférentiellement comprise entre 0,5 et 3.

7. Procédé selon l'une quelconque des revendications précédentes, caractérisé en ce que le nombre de moles de base alcaline mises en oeuvre est de préférence d'au moins 2 fois le stoechiométrie par rapport au nombre de double liaisons et encore plus préférentiallement comprise entre 3 et 5.

8. Procédé selon l'une qualconque des revendications précédentes caractérisé en ce que l'on opère en présence d'un solvant aprotique apolaire ou peu polaire.

9. Procédé selon l'une quelconque des revendications précédentes, caractérisé en ce que la température réactionnelle est comprise entre -20° C et 200° C.

**Patentansprüche**

1. Verfahren zur Herstellung halogenierter Derivate des Cyclopropans bei dem man eine olefinische Verbindung der Formel

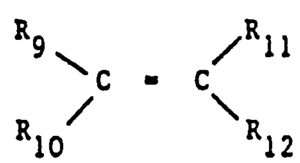

(III)

in der:

. $R_9$ einen Alkyl-, Alkenyl-, Alkoxy-, Aryloxy-, Dialkylamino-, Diarylamino-, Alkylsulfid-, Arylsulfid-, Alkylpolysulfid-, Arylpolysulfid-, Alkylsilan- oder Arylsilanrest mit zwischen 1 und 100 Kohlenstoffatomen darstellt;

.$R_{10}$, $R_{11}$ und $R_{12}$ identisch oder unterschiedlich sind und jeweils ein Wasserstoffatom, einen Alkylrest mit zwischen 1 und 25 Kohlenstoffatomen oder einen Phenylrest darstellen, wobei zwei von diesen Resten derart verknüpft sein können, daß sie einen Kohlenwasserstoffring mit 4 bis 12 Kohlenstoffatomen bilden, oder einen Heterozyklus, der ein Sauerstoffatom oder Stickstoffatom aufweist und zwischen 4 und 7 Kohlenstoffatomen hat

mit einem Halogenderivat reagieren läßt mit der Formel:

$X_1X_2X_3CH$ (I)

in der $X_1$, $X_2$ und $X_3$ identisch oder unterschiedlich sind, und jeweils ein Halogenatom darstellen, wobei mindestens eines ein Chloratom oder Bromatom ist

und mit einer organischen oder mineralischen Alkalibase, dadurch gekennzeichnet, daß die Reaktion durchgeführt wird in Gegenwart eines Maskierungsmittels der allgemeinen Formel:

$N\text{-}[CHR_1\text{-}CHR_2\text{-}O\text{-}(CHR_3\text{-}CHR_4\text{-}O)_n\text{-}R_5]_3$ (II)

in der n eine ganze Zahl größer oder gleich 0 und kleiner oder gleich 10 ist ($0 \leqslant n \leqslant 10$), $R_1$, $R_2$, $R_3$ und $R_4$ identisch oder unterschiedlich sind und jeweils ein Wasserstoffatom oder einen Alkylrest mit 1 bis 4 Kohlenstoffatomen darstellen und $R_5$ einen Alkylrest oder Cycloalkylrest darstellt mit 1 bis 12 Kohlenstoffatomen, einen Phenylrest oder einen $-C_mH_{2m}$-Ø-oder $C_mH_{2m+1}$-Ø-Rest, wobei m zwischen 1 und 12 liegt ($1 \leqslant m \leqslant 12$)

und dadurch, daß das molare Verhältnis des Maskierungsmittels der Formel II zu der durch die Formel (III) definierten Verbindung vorzugsweise kleiner ist als 0,2 und dieses Verhältnis in besonders bevorzugter Weise

zwischen 0,01 und 0,1 liegt.

2. Verfahren nach Anspruch 1, dadurch gekennzeichnet, daß in der Formel II $R_1$, $R_2$, $R_3$ und $R_4$ ein Wasserstoffatom oder einen Methylrest darstellen, wobei $R_5$ und n die vorgenannte Bedeutung haben.

3. Verfahren nach Anspruch 2, dadurch gekennzeichnet, daß in der Formel II n größer oder gleich 0 und kleiner oder gleich 6 ist und $R_5$ einen Alkylrest mit 1 bis 4 Kohlenstoffatomen darstellt.

4. Verfahren nach einem der vorhergehenden Ansprüche, dadurch gekennzeichnet, daß die mineralische Alkalibase der allgemeinen Formel:

$M^+A^-$ (III)

entspricht in der $M^+$ ein Alkalikation darstellt und $A^-$ $OH^-$ oder $NH_2^-$ darstellt.

5. Verfahren nach einem der Ansprüche 1 bis 3, dadurch gekennzeichnet, daß die organische Alkalibase die Formel:

$R_6(O^-)_p(M^+)_p$ (IV)

hat, in der:
- $M^+$ ein Alkalikation darstellt
- p größer oder gleich 1 und kleiner oder gleich 4 ist
- und, wenn p = 1, $R_6$ darstellt:
. einen Alkylrest mit 1 bis 18 Kohlenstoffatomen oder einen Phenylrest, der gegebenenfalls durch einen Alkylrest mit 1 bis 12 Kohlenstoffatomen substituiert ist;
. einen Alkylrest mit 2 bis 5 Kohlenstoffatomen, substituiert wenigstens durch einen OH- oder Alkoxyrest mit 1 bis 4 Kohlenstoffatomen;
. oder einen Rest der Formel:
$R_7$-(O-$R_8$-$)_t$
in der $R_7$ und $R_8$ einen Kohlenwasserstoffrest mit 2 bis 4 Kohlenstoffatomen darstellen und t größer oder gleich 1 und kleiner oder gleich 4 ist;
- wenn p größer oder gleich 2 ist und kleiner oder gleich 4, $R_6$ darstellt:
. einen Kohlenwasserstoffrest, gegebenenfalls substituiert durch mindestens einen OH-Rest, mit 2 bis 5 Kohlenstoffatomen.

6. Verfahren nach einem der vorhergehenden Ansprüche, dadurch gekennzeichnet, daß das Halogenderivat der Formel I und das Alkalihydroxid vorzugsweise in einer Menge entsprechend wenigstens dem 0,2-fachen der stöchiometrischen Menge, bezogen auf die Zahl der Doppelbindungen verwendet werden, in besonders bevorzugter Weise entsprechend dem 0,5 bis 3-fachen der stöchiometrischen Menge.

7. Verfahren nach einem der vorhergehenden Ansprüche, dadurch gekennzeichnet, daß die Anzahl der Mole der eingesetzten Menge der Alkalibase vorzugsweise mindestens dem zweifachen der stöchiometrischen Menge, bezogen auf die Anzahl der Doppelbindungen, entspricht, in besonders bevorzugter Weise dem 3- bis 5-fachen der stöchiometrischen Menge.

8. Verfahren nach einem der vorhergehenden Ansprüche, dadurch gekennzeichnet, daß man in Gegenwart eines aprotischen apolaren oder wenig polaren Lösungsmittels arbeitet.

9. Verfahren nach einem der vorhergehenden Ansprüche, dadurch gekennzeichnet, daß die Reaktionstemperatur zwischen -20° C und 200° C liegt.

## Claims

1. Process for preparing halogenated cyclopropane derivatives, wherein an olefinic compound of formula:

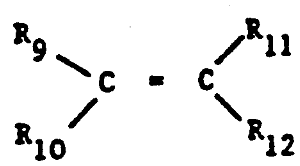

(III)

in which:

$R_9$ denotes an alkyl, alkenyl, alkoxy, aryloxy, dialkylamino, diarylamino, alkyl sulphide, aryl sulphide, alkyl polysulphide, aryl polysulphide, alkylsilane or arylsilane radical having from 1 to 100 carbon atoms; and

$R_{10}$, $R_{11}$ and $R_{12}$, which may be identical or different, denote a hydrogen atom, an alkyl radical having from 1 to 25 carbon atoms or a phenyl radical, it being possible for two of them to be bound so as to form a hydrocarbon ring having from 4 to 12 carbon atoms or a heterocyclic ring containing an oxygen or nitrogen atom and having from 4 to 7 carbon atoms, is reacted with a halogenated derivative of formula:

$X_1X_2X_3CH$ (I)

in which $X_1$, $X_2$ and $X_3$, which may be identical or different, denote a halogen atom, at least one being a chlorine or bromine atom, and with an organic or inorganic alkali metal base, characterized in that the reaction is carried out in the presence of a sequestering agent of general formula:

$N-[CHR_1-CHR_2-O-(CHR_3-CHR_4-O)_n-R_5]_3$ (II)

in which n is an integer greater than or aqual to 0 and less than or equal to 10 ($0 \leqslant n \leqslant 10$), $R_1$, $R_2$, $R_3$ and $R_4$, which may be identical or different, denote a hydrogen atom or an alkyl radical having from 1 to 4 carbon atoms, and $R_5$ denotes an alkyl or cycloalkyl radical having from 1 to 12 carbon atoms, a phenyl radical or a radical $-C_mH_{2m}-Ø$ or $C_mH_{2m+1}-Ø-$, where m is between 1 and 12 ($1 \leqslant m \leqslant 12$), and in that the mole ratio of the sequestering agent of formula (II) to the olefinic compound of formula (III) is preferably less than 0.2 and this ratio is more preferably between 0.01 and 0.1.

2. Process according to Claim 1, characterized in that, in the formula II, $R_1$, $R_2$, $R_3$ and $R_4$ denote a hydrogen atom or a methyl radical, $R_5$ and n-having the above meaning.

3. Process according to Claim 2, characterized in that, in the formula II, n is greater than or equal to 0 and less than or equal to 6, and $R_5$ denotes an alkyl radical having from 1 to 4 carbon atoms.

4. Process according to any one of the preceding claims, characterized in that the inorganic alkali metal base corresponds to the general formula:

$M^+A^-$ (III)

in which $M^+$ denotes an alkali metal cation and $A^-$ denotes $OH^-$ or $NH_2$.

5. Process according to any one of Claims 1 to 3, characterized in that the organic alkali metal base has the formula:

$R_6(O^-)_p(M^+)_p$ (IV)

in which:
$M^+$ denotes an alkali metal cation
p is greater than or equal to 1 and less than or equal to 4
when $p = 1$, $R_6$ denotes:
an alkyl radical having from 1 to 18 carbon atoms or a phenyl radical optionally substituted with an alkyl radical having from 1 to 12 carbon atoms;
an alkyl radical having from 2 to 5 carbon atoms, substituted with at least one OH radical or alkoxy radical having from 1 to 4 carbon atoms:
or a radical of formula:

$R_7-(O-R_8-)_t$

in which $R_7$ and $R_8$ denote a hydrocarbon radical having from 2 to 4 carbon atoms and t is greater than or equal to 1 and less than or equal to 4;
when p is greater than or equal to 2 and less than or equal to 4, $R_6$ denotes:
a hydrocarbon radical optionally substituted with at least one OH radical, and having from 2 to 5 carbon atoms.

6. Process according to any one of the preceding claims, characterized in that the halogenated derivative of formula I and the alkali metal hydroxide are preferably used in amounts equal to at least 0.2 times the stoichiometric amount with respect to the number of double bonds, and still more preferably between 0.5 and 3 times.

7. Process according to any one of the preceding claims, characterized in that the number of moles of alkali metal base employed is preferably at least twice the stoichiometric amount with respect to the number of double bonds, and still more preferably between 3 and 5 times.

8. Process according to any one of the preceding claims, characterized in that the reaction is performed in the presence of an apolar or only slightly polar aprotic solvent.

9. Process according to any one of the preceding claims, characterized in that the reaction temperature is between -20° C and 200° C.